# EUROPÄISCHE PATENTANMELDUNG

(11) **EP 1 306 313 A1**
(43) Veröffentlichungstag der Anmeldung: **02.05.2003**
(21) Anmeldenummer: 01811052.8
(22) Anmeldetag: 27.10.2001
(51) Int. Cl.: B65D 23/12

(54) **Vorrichtung zum Befestigen von Ampullen an einer Injektionsspritze**

(71) Anmelder: Gerner, Zsolt, 8623 Wetzikon (CH)
(72) Erfinder: Gerner, Zsolt, 8623 Wetzikon (CH)
(74) Vertreter: Hammer, Bruno, Dr.

(57) **Zusammenfassung**

Die Vorrichtung zum Befestigen einer oder mehrer Ampullen an einer Injektionsspritze weist Klemm- und Halteteile für die Ampulle, bzw. die Ampullen und die Injektionsspritze auf. Die Klemm- und Halteteile sind miteinander verbunden und bestehen mit Vorteil aus einem Stück. Die Klemmund Halteteile können Spritzgussteile aus federndem Kunststoff sein.

## Beschreibung

Die Erfindung bezieht sich auf eine Vorrichtung zum Befestigen einer oder mehrer Ampullen an einer Injektionsspritze nach dem Oberbegriff des unabhängigen Anspruchs 1 sowie auf eine Anordnung mit einer Injektionsspritze und einer Ampulle nach dem unabhängigen Anspruch.

Bei der Versorgung von Patienten und Pflegebedürftigen werden den Patienten auch Medikamente in Form von Injektionen mit Injektionsspritzen verabreicht. In Spitälern werden die Medikamente, die mit Injektionsspritzen verabreicht werden für eine grössere Zahl von Patienten vorbereitet. Das Medikament wird häufig in einer geschlossenen Ampulle aus Glas angeliefert. Die Ampulle wird geöffnet, indem sie beispielsweise an einer Sollbruchstelle aufgebrochen wird.

Das Vorbereiten einer grösseren Zahl von Injektionen für verschiedene Patienten geschieht nun so, dass eine Ampulle geöffnet wird und deren Inhalt durch die Injektionsnadel der Injektionsspritze in die Injektionsspritze eingesogen oder wie man auch sagt aufgezogen. Dann wird meist die leere Ampulle auf die Injektionsnadel aufgesetzt, so dass die Spitze der Injektionsnadel und wenigstens noch eine Teil der Injektionsnadel sich in der Ampulle befindet. Dies hat den Zweck, den Inhalt der Injektionsspritze mit der leeren Ampulle, die ja bezeichnet ist, zu etikettieren, so dass die Person, welche die Injektion verabreichen wird, erkennen kann welches Medikament oder welche Medikamente sich in welcher Menge in der Injektionsspritze befinden. Weiter schützt die leere Ampulle natürlich auch vor Verletzungen mit der Spitze der Injektionsnadel.

Auch bei grösster Sorgfalt passiert es im alltäglichen Betrieb immer wieder, dass Ampullen von der Injektionsnadel fallen. Wenn dies bei einer Vielzahl von Ampullen und Injektionsspritzen mit verschiedenen Medikamenten passiert, bleibt oft nichts anderes übrig, als die Spritze mit Inhalt wegzuwerfen, um jeder Risiko der Verwechslung von Medikamenten für verschiedene Patienten auszuschliessen. Das nachträgliche Unterscheiden von Medikamenten die injiziert werden, ist auch deshalb sehr schwierig, wenn überhaupt möglich, weil sie sich z.B. farblich fast nie unterscheiden lassen, da sie meist farblos sind. Aber selbst, wenn es gelingt, Ampullen, die ja als Etikette dienen und Injektionsspritzen wieder einander zuzuordnen, bleibt immer ein Gefühl der Unsicherheit beim Pflegepersonal, was das ohnehin stressgeplagte Personal zusätzlich belastet.

Aufgabe der Erfindung ist es ein Vorrichtung zu schaffen, welche zusätzliche und verbesserte Sicherheit schafft. Nach der Erfindung wird die Aufgabe mit einer Vorrichtung gelöst, welche die Merkmale des kennzeichnenden Teils des unabhängigen Anspruchs 1 aufweist. Die abhängigen Ansprüche beziehen sich auf vorteilhafte Ausführungsformen und Weiterbildungen der Erfindung. Die Anordnung nach der Erfindung weist die Merkmale des unabhängigen Anspruchs 8 auf.

Diese Vorrichtung erlaubt es die leere Ampulle als Etikette an der Injektionsspritze mit dem darin aus der Ampulle aufgezogenen Medikament zu auf einfache Art und doch sehr sicher zu befestigen. Die Klemm- und Halteteile sind auf den jeweiligen Querschnitt, bzw. Durchmesser von Ampulle, bzw. Ampullen und Injektionsspritze angepasst. Die mit Vorteil kreisförmigen Öffnungen oder Durchbrüche, in den in welche Ampulle und Injektionsspritze eingesetzt werden können mit Vorteil kreisförmig sein.

Wenn die Klemm- und Halteteile aus einem flexiblen Werkstoff gemacht sind und beispielsweise die Form eines geöffneten Kreises haben, lassen sich sowohl Ampullen als auch Injektionsspritzen leicht einschnappen. Ampullen und Injektionsspritzen haben typisch einen Aussendurchmesser, der im Bereich von 10 mm bis 14 mm liegt. Selbstverständlich gibt es auch Ampullen und Injektionsspritzen, welche kleinere oder grössere Aussendurchmesser aufweisen. Vorrichtungen zum Befestigen für verschieden grosse Ampulle und/oder Injektionsspritze können beispielsweise verschiedene Farbe haben oder mit entsprechend beschriftet sein. Wenn die Vorrichtungen z.B. Spritzgussteile aus Kunststoff sind, ist eine Beschriftung mit Reliefzeichen durch entsprechende Ausgestaltung der Spritzformen besonders einfach zu machen.

Als Werkstoff für die Vorrichtung nach der Erfindung sind z.B. Kunststoffe wie Polyethylen oder Polypropylen, Nylon oder Delrin bestens geeignet. Die Vorrichtung könnte aber auch aus Metall gefertigt sein.

Die Erfindung wird nachstehend anhand der schematischen Zeichnungen, welche Beispiele der Erfindung zeigen näher erläutert.

Es zeigen:
- Fig. 1: in einer Aufsicht eine Vorrichtung nach der Erfindung, welche aus zwei kreisförmigen, geöffneten Ringen;
- Fig. 2: in einer Aufsicht eine weitere Vorrichtung mit zwei kreisförmigen geöffneten Ringen;
- Fig. 3.: Vorrichtung nach der Erfindung mit Ampulle und Injektionsspritze.

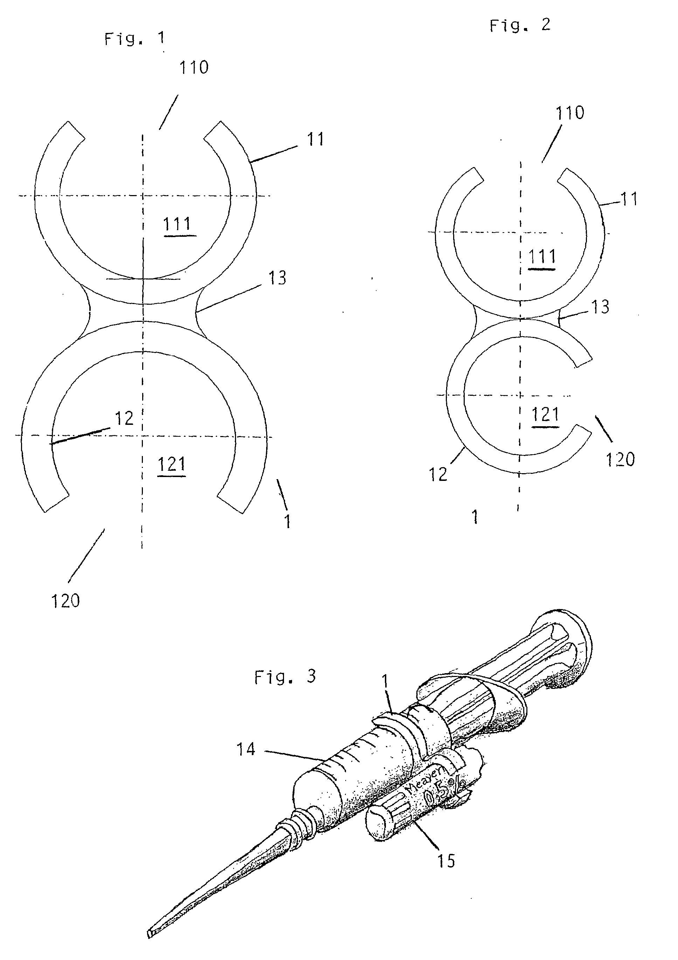

Die Vorrichtung zum Befestigen 1 von Fig. 1 besteht im wesentlichen aus den beiden Ringen 11 und 12, welche je eine Öffnung 110, bzw. 120 aufweisen Die beiden Ringe sind mit einem brückenartigen Verbindungsstück 13 verbunden. Ampulle und Injektionsspritze können durch die Öffnungen 110 bzw. 120 in den Durchbruch 111, bzw. 121 gegen das Verbindungsstück 13 hin, eingeschnappt werden. Ampulle und Injektionsspritze können aber auch in den Durchbruch 111, bzw. 121 eingeschoben werden. Die beiden Öffnungen 110 und 120 sind in diesem Beispiel entgegengesetzt angeordnet. Bei der in Fig. 2 gezeigten Vorrichtung zum Befestigen 1 hingegen sind die Öffnungen 110 und 120 rechtwinklig zueinander angeordnet.

Die Vorrichtung zum Befestigen kann auch einen Ring für die Injektionsspritze mit zwei oder mehr Ringen für Ampullen aufweisen. Genau so kann die Vorrichtung zum Befestigen z.B. einen geschlossenen Ring und einen Ring mit Öffnung aufweisen. Die Vorrichtung zum Befestigen braucht auch nicht aus ringförmigen Elementen zu bestehen. Elemente mit beliebiger Form können kreisförmige oder sonst wie geformte Durchbrüche mit oder ohne Öffnungen aufweisen.

Fig. 3 schliesslich zeigt eine Injektionsspritze 14 und eine Ampulle 15, welche mit einer Vorrichtung zum Befestigen 1 einander zugeordnet sind, bzw. mit einander verbunden sind. Die Ampulle 15 ist die Etikette an der Injektionsspritze 14 und beschreibt, welches Medikament in welcher Menge und Konzentration sich in der Injektionsspritze 14 befindet.

Die Vorrichtung zum Befestigen einer oder mehrer Ampullen an einer Injektionsspritze weist Klemm- und Halteteile für die Ampulle, bzw. die Ampullen und die Injektionsspritze auf. Die Klemm- und Halteteile sind miteinander verbunden und bestehen mit Vorteil aus einem Stück. Die Klemmund Halteteile können Spritzgussteile aus federndem Kunststoff sein.

## Patentansprüche

1. Vorrichtung (1) zum Befestigen einer oder mehrer Ampullen (15) an einer Injektionsspritze (14) **gekennzeichnet durch** Klemm- und Halteteile (11, 12) für die Ampulle (15) oder die Ampullen und für die Injektionsspritze (14) und bei der die Klemm- und Halteteile miteinander verbunden sind.

2. Vorrichtung (1) nach Anspruch 1, mit einem Verbindungsstück (13) zwischen den Klemm- und Halteteilen (1).

3. Vorrichtung (1) nach Anspruch 1 bei welcher die aus einem elastischen Werkstoff aus einem flexiblen Werkstoff.

4. Vorrichtung (1) nach einem der Ansprüche 1 bis 3, bei welcher wenigstens eines der Klemm- und Halteteile (11, 12) einen kreisartigen Durchbruch (111, 121), mit gleichem oder unterschiedlichem Innendurchmesser für die Aufnahme der Injektionsspritze (14) und/oder der Ampulle(n) (15) aufweist.

5. Vorrichtung (1) nach einem der Ansprüche 1 bis 4, bei welcher wenigstens eines der Klemm- und Halteteile (11, 12) einen Durchbruch (111; 121) mit einer Öffnung (110; 120) aufweist, so dass Ampulle (15) und/oder Injektionsspritze (14) vom Klemm- und Halteteil (11; 12) nur teilweise umfasst wird.

6. Vorrichtung (1) nach einem der Ansprüche 1 bis 5, bei welcher die Öffnung (110; 120) im Durchbruch (111; 121) in den Klemm- und Halteteilen (11; 12) entgegengesetzt, in einem rechten Winkel oder in beliebigem Winkeln zueinander angeordnet sind.

7. Vorrichtung (1) nach einem der Ansprüche 1 bis 6, die aus Kunststoff, vorzugsweise einem Thermoplast wie Polyethylen, Polypropylen, oder aus einem Metall gefertigt ist.

8. Anordnung mit einer Vorrichtung (1) nach einem der Ansprüche 1 bis 7, und mit einer Injektionsspritze (14) sowie einer Ampulle (15) in je einem der Klemm- und Halteteile (11; 12).
